# EUROPÄISCHE PATENTSCHRIFT

(11) **EP 0 680 319 B1**
(45) Veröffentlichungstag und Bekanntmachung des Hinweises auf die Patenterteilung: **12.03.1997**
(21) Anmeldenummer: 94904650.2
(22) Anmeldetag: 10.01.1994
(51) Int. Cl.: A61K 31/21, A61K 9/70

(54) **NITROGLYCERINHALTIGES PFLASTER, VERFAHREN ZU SEINER HERSTELLUNG UND VERWENDUNG**
NITROGLYCERINE-CONTAINING PLASTER, PROCESS FOR PRODUCING THE SAME AND ITS USE
PANSEMENT CONTENANT DE LA NITROGLYCERINE, PROCEDE DE FABRICATION ET UTILISATION

(30) Priorität: 23.01.1993 DE 4301781
(43) Veröffentlichungstag der Anmeldung: 08.11.1995
(73) Patentinhaber: LTS LOHMANN Therapie-Systeme GmbH, 56567 Neuwied (DE)
(72) Erfinder: MECONI, Reinhold, D-56567 Neuwied (DE); RADEMACHER, Tina, D-53604 Bad Honnef (DE)
(74) Vertreter: Flaccus, Rolf-Dieter, Dr.
(86) Internationale Anmeldenummer: EP9400053
(87) Internationale Veröffentlichungsnummer: WO9416691

(56) Entgegenhaltungen:
- EP-A- 0 204 968
- EP-A- 0 251 425
- EP-A- 0 305 758
- EP-A- 0 384 267
- DE-A- 3 315 245

## Beschreibung

Die Erfindung betrifft ein wirkstoffhaltiges Pflaster zur kontrollierten Abgabe von Nitroglycerin an die Haut, bestehend aus einer undurchlässigen Rückschicht, einem damit verbundenen Haftschmelzklebner und Wirkstoff enthaltenden Reservoir sowie einer wiederablösbaren Schutzschicht, dadurch gekennzeichnet, daß das Reservoir ein lösemittelfreies Homogenisat aus:
a) klebrigmachenden Weich- und/oder Hartharzen,
b) Weichmacher
c) einer Nitroglycerin-Zubereitung ist ,
jedoch keine anderen Polymere enthält.

Nitroglycerinpflaster sind bereits bekannt. Die Herstellung von Nitroglycerinpflastern ist insofern problematisch, als Nitroglycerin explosiv ist und infolgedessen seine Verdampfung sowie thermische und mechanische Belastungen möglichst vermieden werden müssen.

Es ist bevorzugt, bei möglichst niedrigen Temperaturen, insbesondere bei Raumtemperatur, zu arbeiten, wobei häufig die nitroglycerinhaltige Haftkleberschicht aus einer Lösung hergestellt wird. Die derart hergestellten bekannten pflasterartigen transdermalen therapeutischen Systeme zur Abgabe von Nitroglycerin erfüllten durchaus die therapeutischen Aufgaben, sind aber in der Herstellung zu aufwendig.

In der DE-OS 3222800 (ALZA) ist ein Nitroglycerinpflaster beschrieben, wobei als nitroglycerinhaltige Matrix eine bei Raumtemperatur durch Andickung einer Nitroglycerinlösung mit einem rheologischen Mittel erhältliche nicht klebende viskose Masse eingesetzt wird. Auch im US-Patent 3742951 (CIBA-GEIGY) sowie der DP-PS 3315272 und DE-OS 3315245 (LOHMANN/SCHWARZ) werden einfach aufgebaute Nitroglycerinpflaster mit bei Raumtemperatur aus Lösung hergestellten haftklebenden Matrixmaterialien beschrieben. Es ist auch bereits bekannt, z.B. aus der DE-OS 3642931 (CIBA-GEIGY), mehrteilige Nitroglycerinreservoirs einzusetzen.

Aus der EP-A2-175562 sind allgemein Haftschmelzkleber bekannt; dort ist aber keine Anregung dahingehend gegeben, ob und wie diese für Wirkstoffreservoirs, insbesondere solche, die Nitroglycerin enthalten, ausgewählt werden müssen.

Gattungsgemäße Nitroglycerinpflaster sind in der EP-A 0204968 beschrieben, wobei dort ein selbstklebendes Reservoir aus Kautschuk und klebrigmachendem Harz mit Nitroglycerin eingesetzt wird. Dieses haftklebende Reservoir wird aus Lösung hergestellt, so daß hier also das Problem des Absaugens von Lösemittelresten und ggf. auch der damit erfolgenden Abdampfung von Nitroglycerin auftritt.

Die Verwendung von Lösemitteln bei der Haftkleberschichtherstellung ist aus mehreren Gründen nachteilig. Die Herstellung der Lösungen erfordert einen hohen technischen Aufwand, da die Geräte explosionsgeschützt sein müssen. Für medizinische Zwecke müssen hochreine und damit teure Lösemittel für die Auflösung der Kleber bzw. deren Ausgangsmaterialien eingesetzt werden. Ein weiteres Problem besteht darin, Lösemittelfreiheit im Pflaster zu erreichen, wofür teure Trocknungs und Absauganlagen erforderlich sind. Zusätzlich treten Kosten durch Wiedergewinnung und Abscheidung der Lösemittel auf, um Umweltbelastung zu vermeiden. Daneben stellt die Brennbarkeit der Lösemittel, insbesondere beim hier vorliegenden explosiven Wirkstoff ein zusätzliches Risiko dar. Die meisten organischen Lösemittel sind ferner schädlich für den menschlichen Organismus, so daß aufwendige Schutzmaßnahmen für die im Betrieb tätigen Personen getroffen werden müssen.

Aus der DE-3743946 ist ein Nitroglycerinpflaster bekannt, das durch Beschichten eines nitroglycerinhaltigen Klebers aus der Schmelze bei 40 - 80°C hergestellt wird.

Die Praxis hat jedoch gezeigt, daß die Beschichtung mit großen Schwierigkeiten verbunden ist wegen der hohen Viskosität der Beschichtungsmasse. Durch die im nitroglycerinhaltigen Kleber enthaltenen polymeren Verbindungen (z.B. Ethylen-Vinylacetat-Copolymere) ist die Viskosität so hoch, daß die Herstellung der nitroglycerin-haltigen Pflaster auf äußerst große Probleme stößt.

Es ist somit Aufgabe der Erfindung, die oben angeführten Nachteile zu vermeiden und einen solchen Haftschmelzkleber zur Verfügung zu stellen, der eine einwandfreie Verarbeitung im technischen Maßstab ermöglicht.

Die Lösung der Aufgabe besteht darin, unter Verzicht auf polymere Verbindungen durch die alleinige Verwendung von Harzen zu verarbeitbaren Schmelzen zu kommen.

Hier bietet sich ein großer Spielraum für die Formulierung des Reservoirs auf der Basis von Schmelzhaftklebern. So ist es möglich, die Nitroglycerin-Zubereitung mit verschiedenen Weichharzen abzumischen. Darüberhinaus bestehen auch Möglichkeiten, verschiedene Hartharze oder auch die Kombination aus Hart- und Weichharzen zu verwenden.

Im Sinne der Erfindung sind Weichharze klebrigmachende Harze, die bei Raumtemperatur halbfest oder flüssig sind bzw. bis ca. 60°C schmelzen. Dazu zählen z.B. Methylester von Harzsäuren, Methylester von hydrierten Harzsäuren, Triethylenglycolester von hydrierten Harzsäuren, Hydroabietylalkohol, Phthalsäureester von Hydroabietylakohol, aromatische Kohlenwasserstoffharze, niedermolekulare Styrolharze und hydrierte Kohlenwasserstoffharze.

Hartharze im Sinne der Erfindung sind klebrigmachende Harze, die bei Raumtemperatur fest sind und ab ca. 60°C erweichen. Dazu zählen modifizierte Harzsäuren, hydrierte und nicht hydrierte Harzester polymerisierte Harzester säuremodifizierte Harzester aromatische und aliphatische Kohlenwasserstoffharze, Alpha-Methylstyrolharze, Alpha-Methylstyrol/Vinyltoluol-Copolymer-Harze und hydrierte Kohlenwasserstoffharze.

Der Anteil an klebrigmachenden Harzen in der nitroglycerinhaltigen Klebmasse beträgt 10-43 Gew.-%.

Das nitroglycerin-haltige Reservoir enthält zusätzlich Weichmacher. Geeignete Weichmacher sind dem Fachmann bekannt und beispielsweise in der DE-3743946 beschrieben. Das nitroglycerinhaltige Reservoir enthält üblicherweise Weichmacher in einem Anteil von 0,1-5 Gew.-%.

Üblicherweise sind im wirkstoffhaltigen Reservoir auch Alterungsschutzschmittel in einer Konzentration von 0,1 - 2 Gew.-% enthalten. Diese sind dem Fachmann bekannt und z.B. in der DE-3743946 beschrieben.

Die Materialien für die undurchlässige Rückschicht und die wiederablösbare Schutzschicht sind dem Fachmann ebenso bekannt (z.B. DE-3843239).

Ein besonders bevorzugter Aufbau des transdermalen Nitroglycerinpflasters ist das Matrixsystem, bei dem bekanntlich die Matrix die Steuerung für die Wirkstofffreisetzung übernimmt und diese dem √ t-Gesetz nach Higuchi gehorcht. Das bedeutet jedoch nicht, daß in besonderen Fällen auch das Membransystem angezeigt ist. Hierbei ist zwischen Reservoir und Haftkleberschicht eine die Nitroglycerin-Freisetzung steuernde Membran angebracht.

Für den Fall, daß das Reservoir keine ausreichende Eigenklebrigkeit zur Haut aufweist, kann dieses mit einer Haftkleberschicht versehen werden. Auf diese Weise ist gewährleistet, daß das transdermale Pflaster über den gesamten Applikationszeitraum auf der Haut haftet.

Die Dicke des transdermalen Pflasters richtet sich nach den therapeutischen Erfordernissen und kann entsprechend angepaßt werden. Sie liegt üblicherweise im Bereich von 0,03-0,4 mm.

Im Falle nicht ausreichender Eigenklebrigkeit zur Haut besteht eine weitere Möglichkeit, das Reservoir auf der Haut zu fixieren darin, das Reservoir mit einem haftklebenden Rand zu versehen.

Die Herstellung des transdermalen Pflasters stellt sich besonders einfach dar. Die Harze werden aufgeschmolzen, die übrigen Reservoir-Bestandteile bei einer Temperatur von weniger als 55°C zugegeben und durch Kneten oder Rühren homogenisiert. Es resultieren Reservoir-Massen mit einer Viskosität von 1320 - 8253 dPa·s. Die so erhaltene nitroglycerinhaltige Reservoir-Masse wird auf die wiederablösbare Schutzschicht aufgetragen und die undurchlässige Rückschicht zukaschiert.

Die zur Herstellung des transdermalen Pflasters erforderliche Maschine hat nur einen geringen Platzbedarf und ist deutlich kostengünstiger als übliche Beschichtungsanlagen zur Verarbeitung von lösemittelhaltigen Klebern. Sie arbeitet darüberhinaus mit einer hohen Geschwindigkeit im Bereich von ca. 10-50 m pro Minute.

### Beispiel 1

- 11,05 g: Weichharz (Hydroabietylalkohol: Handelsname Abitol der Fa. Hercules),
- 28,74 g: Hartharz (aliphatisches Kohlenwasserstoffharz: Hercures C der Fa. Hercules) und
- 2,29 g: Weichmacher (mittelkettige Triglyceride: Miglyol 812 der Fa. Dynamit Nobel)
werden bei 120°C durch Kneten homogenisiert. Die Schmelze wird auf 55°C abgekühlt und anschließend werden 57,47 g Nitroglycerin-Lactose-Zubereitung (10% Nitroglycerin) zugegeben und durch Kneten homogenisiert.

Die so erhaltene wirkstoffhaltige Reservoir-Masse (Viskosität: 8253 dPa·s) wird bei 55°C auf die wiederablösbare Schutzschicht (Hostaphan RN 100 einseitig mit Silikon beschichtet (100 µm dick) - Fa. Kalle) so beschichtet, daß das Reservoir mit einem Flächengewicht von 175,5 g/m² resultiert. Darauf wird die undurchlässige Rückschicht (Hostaphan RN 36 (36 µm dick) - Fa. Kalle) kaschiert.

Aus dem so erhaltenen Laminat werden 16 cm² große Pflaster mit abgerundeten Ecken gestanzt.

### Beispiel 2

Die Herstellung erfolgt wie in Beispiel 1 beschreiben, jedoch nach der in Tabelle 1 angegebenen Herstellformel; Viskosität: 3301 dPa·s bei 55°C).

### Beispiel 3

Die Herstellung erfolgt wie in Beispiel 1 angegeben, jedoch nach der in Tabelle 1 angegebenen Herstellformel (Viskosität: 1320 dPa·s bei 55°C).

### Analytik

Die Wirkstofffreisetzung der 16 cm² großen transdermalen Pflaster wird nach der in der USP XXII beschriebenen Rotating bottle-Methode in 0,9%iger Kochsalzlösung bei 37°C bestimmt.
Die Ergebnisse sind in Tabelle 2 dargestellt.

**Tabelle 1**

| *Herstellformel* | | | | | |
|---|---|---|---|---|---|
| Beispiel | Abitol (g) | Hercures C (g) | Miglyol 812 (g) | Nitroglycerin-Lactose (10%ig) (g) | Flächengewicht des Reservoirs (g/m²) |
| 1 | 11,50 | 28,74 | 2,29 | 57,47 | 175,5 |
| 2 | 16,10 | 24,13 | 2,30 | 57,47 | 172,0 |
| 3 | 20,11 | 20,11 | 2,29 | 57,50 | 175,0 |

**Tabelle 2**

| *Wirkstofffreisetzung* | | | | |
|---|---|---|---|---|
| Beispiel | Wirkstofffreisetzung (mg Nitroglycerin / 16 cm²) nach | | | |
| | 2 Std. | 4 Std. | 6 Std. | 24 Std. |
| 1 | 2,15 | 3,88 | 6,08 | 10,87 |
| 2 | 5,50 | 7,19 | 8,60 | 10,26 |
| 3 | 6,85 | 9,76 | 11,71 | 15,67 |

## Patentansprüche

1. Wirkstoffhaltiges Pflaster zur kontrollierten Abgabe von Nitroglycerin an die Haut, bestehend aus einer undurchlässigen Rückschicht, einem damit verbundenen Haftschmelzklebner und Wirkstoff enthaltenden Reservoir sowie einer wiederablösbaren Schutzschicht, dadurch gekennzeichnet, daß das Reservoir ein lösemittelfreies Homogenisat aus:
a) klebrigmachenden Weich- und/oder Hartharzen,
b) Weichmacher
c) einer Nitroglycerin-Zubereitung ist ,
jedoch keine anderen Polymere enthält.

2. Wirkstoffhaltiges Pflaster nach Anspruch 1, dadurch gekennzeichnet, daß die klebrigmachenden Harze Weichharze sind.

3. Wirkstoffhaltiges Pflaster nach Anspruch 1, dadurch gekennzeichnet, daß die klebrigmachenden Harze Hartharze sind.

4. Wirkstoffhaltiges Pflaster nach Ansprüchen 1 - 3, dadurch gekennzeichnet, daß die klebrigmachenden Harze aus einem Gemisch von Weich- und Hartharzen bestehen.

5. Wirkstoffhaltiges Pflaster nach Ansprüchen 1 - 4, dadurch gekennzeichnet, daß der Anteil an klebrigmachenden Harzen zwischen 10 und 43 Gew.-% liegt.

6. Wirkstoffhaltiges Pflaster nach Ansprüchen 1 - 5, dadurch gekennzeichnet, daß das Reservoir 0,3 - 10,0 Gew.-% Nitroglycerin enthält.

7. Wirkstoffhaltiges Pflaster nach den Ansprüchen 1 - 6, dadurch gekennzeichnet, daß das Nitroglycerin den vorgemischten Harzen in einer Nitroglycerin-Lactose-Zubereitung hinzugemischt ist.

8. Wirkstoffhaltiges Pflaster nach Ansprüchen 1 - 7, dadurch gekennzeichnet, daß das Reservoir 0, 1 - 5 Gew.-% Weichmacher enthält.

9. Wirkstoffhaltiges Pflaster nach Ansprüchen 1 - 8, dadurch gekennzeichnet, daß das Reservoir 0,1 - 2 Gew.-% Alterungsschutzmittel enthält.

10. Wirkstoffhaltiges Pflaster nach Ansprüchen 1 - 9, dadurch gekennzeichnet, daß das Reservoir eine Dicke von 0,03 - 0,4 mm aufweist.

11. Wirkstoffhaltiges Pflaster nach Ansprüchen 1- 10, dadurch gekennzeichnet, daß das Reservoir aus mehreren Schichten besteht.

12. Wirkstoffhaltiges Pflaster nach Ansprüchen 1 - 11, dadurch gekennzeichnet, daß das Reservoir im Falle unzureichender Eigenklebrigkeit zur Haut hin mit einer Haftkleberschicht versehen ist.

13. Wirkstoffpflaster nach Ansprüchen 1 - 12, dadurch gekennzeichnet, daß das Reservoir im Falle unzureichender Eigenklebrigkeit zur Haut mit einem haftklebenden Rand versehen ist.

14. Verfahren zur Herstellung eines nitroglycerinhaltigen Pflasters, gemäß Anspruch 1 gekennzeichet durch die Arbeitsschritte:
a) Weichharz und/oder Hartharz und Weichmacher werden bei erhöhten Temperaturen ohne Verwendung von Lösemittel in einer Schmelze durch Kneten homogenisiert;
b) die Schmelze wird auf weniger als 60°C abgekühlt, anschließend wird eine Nitroglycerin-Zubereitung zugegeben und durch weiteres kneten homogenisiert;
c) die so erhaltene wirkstoffhaltige Reservoir-Masse wird bei gleichbleibend niedriger Temperatur auf die wiederablösbare Schutzschicht beschichtet und darauf wird die undurchlässige Rückschicht kaschiert;
d) aus dem so erhaltenen Laminat werden Pflaster ausgestanzt.

15. Nitroglycerinhaltige Pflaster nach Ansprüchen 1 - 13 zur Anwendung für therapeutische Zwecke in der Human- und Veterinärmedizin.

## Claims

1. An active substance-containing patch for the controlled release of nitroglycerin to the skin, consisting of an impermeable backing layer, a reservoir bonded thereto and comprising pressure-sensitive hot melt adhesive and active substance, and a removable protective layer, characterized in that the reservoir comprises a solvent-free homogenized mixture comprising:
a) tackifying soft and/or hard resins,
b) plasticizers,
c) nitroglycerin preparation,
but does not contain any other polymers.

2. The active substance-containing patch according to claim 1 characterized in that the tackifying resins are soft resins.

3. The active substance-containing patch according to claim 1 characterized in that the tackifying resins are hard resins.

4. The active substance-containing patch according to claims 1 - 3 characterized in that the tackifying resins consist of a mixture of soft and hard resins.

5. The active substance-containing patch according to claims 1 - 4 characterized in that the proportion of tackifying resins ranges between 10 and 43% by weight.

6. The active substance-containing patch according to claims 1 - 5 characterized in that the reservoir comprises 0.3 - 10.0% by weight nitroglycerin.

7. The active substance-containing patch according to claims 1 - 6 characterized in that the nitroglycerin has been mixed to the pre-mixed resins in a nitroglycerin-lactose-preparation.

8. The active substance-containing patch according to claims 1 - 7 characterized in that the reservoir comprises 0.1 - 5% by weight plasticizers.

9. The active substance-containing patch according to claims 1 - 8 characterized in that the reservoir comprises 0.1 - 2% by weight anti-ageing agents.

10. The active substance-containing patch according to claims 1 - 9 characterized in that the reservoir has a thickness of 0.03 - 0.4 mm.

11. The active substance-containing patch according to claims 1 - 10 characterized in that the reservoir consists of several layers.

12. The active substance-containing patch according to claims 1 - 11 characterized in that the reservoir is provided with a pressure-sensitive adhesive layer if it fails to exhibit sufficient self-tackiness to the skin.

13. The active substance-containing patch according to claims 1 - 12 characterized in that the reservoir is provided with a pressure-sensitive adhesive edge if it fails to exhibit sufficient self-tackiness to the skin.

14. A process for the production of a nitroglycerin-containing patch according to claim 1, characterized by the steps:
a) soft resin and/or hard resin and softener(s) are homogenized in a melt by kneading at elevated temperatures, without the use of solvents;
b) the melt is cooled down to less than 60 °C and subsequently a nitroglycerin preparation is added; this is homogenized by further kneading;
c) the thus-obtained active substance-containing reservoir mass is coated onto the removable protective layer at a constant low temperature and the impermeable backing layer is laminated thereon;
d) patches are punched out of the thus-obtained laminate.

15. Nitroglycerin-containing patches according to claims 1-13, to be used for therapeutic purposes in human and veterinary medicine.

## Revendications

1. Emplâtre ou timbre contenant un principe actif pour la libération réglée de nitroglycérine dans la peau, qui se compose d'une couche dorsale imperméable d'un réservoir contenant le principe actif et une colle fusible de contact qui y adhère, ainsi qu'une couche protectrice amovible, caractérisé en ce que le réservoir contient un homogénéisat dépourvu de solvant et constitué de :
a) des résines molles et/ou dures conférant de l'adhésivité,
b) des plastifiants,
c) une préparation de nitroglycérine,
mais ne contient cependant pas d'autres polymères.

2. Timbre contenant un principe actif suivant la revendication 1, caractérisé en ce que les résines conférant de l'adhésivité sont des résines molles.

3. Timbre contenant un principe actif suivant la revendication 1, caractérisé en ce que les résines conférant de l'adhésivité sont des résines dures.

4. Timbre contenant un principe actif suivant l'une quelconque des revendications 1 à 3, caractérisé en ce que les résines conférant de l'adhésivité se composent d'un mélange de résines molles et de résines dures.

5. Timbre contenant un principe actif suivant l'une quelconque des revendications 1 à 4, caractérisé en ce que la proportion de résines conférant de l'adhésivité varie de 10 à 43% en poids.

6. Timbre contenant un principe actif suivant l'une quelconque des revendications 1 à 5, caractérisé en ce que le réservoir contient de 0,3 à 10,0% en poids de nitroglycérine.

7. Timbre contenant un principe actif suivant l'une quelconque des revendications 1 à 6, caractérisé en ce que la nitroglycérine est incorporée par mélange aux résines préalablement mélangées dans une préparation de nitroglycérine-lactose.

8. Timbre contenant un principe actif suivant l'une quelconque des revendications 1 à 7, caractérisé en ce que le réservoir contient de 0,1 à 5% en poids de plastifiants.

9. Timbre contenant un principe actif suivant l'une quelconque des revendications 1 à 8, caractérisé en ce que le réservoir contient de 0,1 à 2% en poids d'agents de protection contre le vieillissement.

10. Timbre contenant un principe actif suivant l'une quelconque des revendications 1 à 9, caractérisé en ce que le réservoir possède une épaisseur de 0,03 à 0,4 mm.

11. Timbre contenant un principe actif suivant l'une quelconque des revendications 1 à 10, caractérisé en ce que le réservoir se compose de plusieurs couches.

12. Timbre contenant un principe actif suivant l'une quelconque des revendications 1 à 11, caractérisé en ce que, dans le cas d'un caractère autocollant à la peau insuffisant, le réservoir est pourvu d'une couche de colle de contact.

13. Timbre contenant un principe actif suivant l'une quelconque des revendications 1 à 12, caractérisé en ce que, dans le cas d'un caractère autocollant à la peau insuffisant, le réservoir est pourvu d'un bord autocollant.

14. Procédé de préparation d'un timbre contenant de la nitroglycérine suivant la revendication 1, caractérisé en ce qu'il comprend les étapes opératoires consistant à :
a) homogénéiser la résine molle et/ou la résine dure et le plastifiant, à des températures élevées et sans recourir à l'emploi d'un solvant, dans une masse fondue par pétrissage ou malaxage;
b) refroidir la masse fondue jusqu'à une température inférieure à 60°C, ensuite ajouter une préparation, à base de nitroglycérine et homogénéiser le tout par pétrissage ou malaxage poursuivi;
c) appliquer la masse constituant le réservoir et comprenant le principe actif ainsi obtenue à basse température constante sur la couche protectrice amovible et y lamifier la couche dorsale imperméable;
d) découper des timbres par estampage hors du lamifié ainsi obtenu.

15. Timbre contenant de la nitroglycérine suivant les revendications 1 à 13, à utiliser à des fins thérapeutiques en médecine humaine et vétérinaire.
